# EUROPÄISCHE PATENTSCHRIFT

(11) **EP 0 582 245 B1**
(45) Veröffentlichungstag und Bekanntmachung des Hinweises auf die Patenterteilung: **19.11.1997**
(21) Anmeldenummer: 93112339.2
(22) Anmeldetag: 02.08.1993
(51) Int. Cl.: A61K 7/50, A61K 7/48

(54) **Badezusatzpräparat**
Bath additive composition
Additif pour bain

(30) Priorität: 07.08.1992 DE 4226173
(43) Veröffentlichungstag der Anmeldung: 09.02.1994
(73) Patentinhaber: Solvay Alkali GmbH, 30173 Hannover (DE)
(72) Erfinder: Jakobson, Gerald, D-4134 Rheinberg 2 (DE); Siemanowski, Werner, D-4134 Rheinberg 1 (DE); Uhlig, Karl-Heinz, D-4150 Krefeld-Traar (DE)
(74) Vertreter: Lauer, Dieter, Dr.

(56) Entgegenhaltungen:
- EP-A- 0 582 246
- DE-A- 4 005 819
- US-A- 5 045 337
- S.T.N., File Supplier, KARLSRUHE, DE, File Chemical Abstracts, vol 108, n 114726 & JP-A-62266135( Pola Chemical Ind.) * Zusammenfassung *

## Beschreibung

Die vorliegende Erfindung betrifft ein Badezusatzpräparat, insbesondere Ölbadpräparat, enthaltend mindestens eine grenzflächenaktive Komponente, vorzugsweise einen wasserlöslichen Emulgator, mindestens eine ölige oder ölhaltige Komponente, ausgewählt aus den natürlichen und synthetischen, mineralischen, ätherischen und fetten tierischen und pflanzlichen Ölen, gegebenenfalls ein Lösemittel oder Lösemittelgemisch sowie gegebenenfalls weitere Hilfs- und Zusatzstoffe.

Wannenbäder unter Zusatz geeigneter kosmetischer und/oder therapeutischer bzw. physiologisch wirkender Badezubereitungen (Badezusatzpräparate) erfreuen sich zunehmender Beliebtheit, da sie nicht nur zur Körperreinigung dienen, sondern durch ihre pflegenden Bestandteile einen regenerierenden und normalisierenden Effekt auf der Haut bewirken und durch ihre medizinisch-therapeutischen Zusätze, wie beispielsweise ätherische Öle, einen erfrischenden, entspannenden und gegebenenfalls heilenden Einfluß auf den menschlichen Organismus haben. Derartige Badezubereitungen werden auch als Öl- oder Pflegebäder bezeichnet. Darüber hinaus können sie auch parfümierende Zusätze enthalten.

Derartige Badezubereitungen werden in fester, flüssiger (einschließlich gelartiger und hochviskoser) oder pastöser Form angeboten. Bevorzugt werden flüssige Zubereitungen eingesetzt, die häufig in Form wäßriger oder öliger Emulsionen oder Dispersionen vorliegen. Sie enthalten üblicherweise folgende Hauptbestandteile: mindestens eine oberflächen- beziehungsweise grenzflächenaktive Verbindung, die als Tensid, Emulgier-, Dispergier-, Solubilisierungs- und/oder Netzmittel wirkt und entweder vorwiegend wasser- oder vorwiegend fettlöslich ist, mindestens einen Wirkstoff, der einen pflegenden, schützenden, regenerierenden, vitalisierenden und/oder medizinisch-therapeutischen Effekt ausübt sowie gegebenenfalls ein Lösemittel oder Lösemittelgemisch auf wäßriger oder organischer Basis.

Rein kosmetische Wirkstoffe sind beispielsweise Zusätze auf Lipidbasis und andere cremende, rückfettende, das Wasserbindungsvermögen der Haut beeinflussende Stoffe. Hierzu zählen auch bestimmte pflanzliche und tierische Öle, die kosmetische Eigenschaften aufweisen. Ein medizinisch-therapeutischer Einfluß auf den menschlichen Organismus kann beispielweise mit Hilfe von speziellen ätherischen Ölen oder Pflanzenextrakten erreicht werden.

Als weitere Bestandteile können Badezusatzpräparate Schaumverbesserer und -verstärker, pH-Wert-Regulatoren, Konservierungsmlttel mit antiseptischen, bakteriziden oder bakteriostatischen Eigenschaften, Antioxidationsmittel, Verdicker bzw. Viskositätsregler, Farbstoffe und/oder Duftstoffe enthalten.

Die Badezubereitungen werden gemäß Dosierungsangabe dem Badewasser zugegeben, wobei das Präparat derart eingestellt ist, daß eine gleichmäßige Verteilung der Formulierungsbestandteile im Badewasser gewährleistet ist oder es zu einer Spreitung auf der Wasseroberfläche kommt.

Badezubereitungen gemäß dem vorgenannten Stand der Technik weisen jedoch in zweierlei Hinsicht Nachteile auf.

Einerseits bereitet die Einarbeitung öliger und ölhaitiger Zusätze, insbesondere natürlicher oder naturidentischer ätherischer oder fetter pflanzlicher Öle, in kosmetische und pharmazeutische Zubereitungen Schwierigkeiten, da sich derartige Stoffe naturgemäß nur schwer emulgieren lassen.

Daher müssen hier besondere Emulgatoren oder Emulgatorgemische eingesetzt werden. Im Stand der Technik sind dies bisher vor allem Ethylenoxid-Anlagerungsverbindungen, die zwar gute emulgierende Eigenschaften besitzen, jedoch wegen ihrer toxikologischen und dermatologischen Eigenschaften weniger wünschenswert sind und insbesondere keine kosmetischen Effekte ausüben können.

Andererseits weisen die bekannten Badezubereitungen meist eine aufwendige, häufig auch kostspielige Rezeptur auf, wenn sie als Kombinationspräparat sowohl eine kosmetische als auch eine medizinisch-therapeutische Wirksamkeit besitzen sollen.

Zudem sind Emulgatoren, die nicht nur zur Herstellung stabiler O/W-Systeme, wie beispielsweise Ölbadformulierungen, geeignet sind, sondern darüber hinaus auch eine physiologische Wirkung, insbesondere einen hautpflegenden und -schützenden Effekt ausüben, bisher nur in unzureichendem Maße verfügbar.

Aufgabe der vorliegenden Erfindung war es daher, ein Badezusatzpräparat, insbesondere ein Ölbadpräparat bereitzustellen, das einen kosmetischen, insbesondere einen hautpflegenden, -schützenden und -regenerierenden und/oder einen therapeutischen Effekt auf den menschlichen Organismus ausübt und zugleich eine einfache Rezeptur aufweist, wobei ein wasserlöslicher, flüssiger Emulgator eingesetzt werden sollte, der keine toxikologischen oder dermatologischen Probleme aufwirft, sondern gesundheitlich völlig unbedenklich ist und insbesondere hautpflegende Eigenschaften besitzt.

Daher sollte die Badezubereitung als grenzflächenaktiven Bestandteil mindestens einen Polyglycerinfettsäureester enthalten. Derartige Verbindungen bzw. Gemische von Polyglycerinfettsäureestern werden bereits als Solubilisatoren und Emulgatoren bzw. Dispergierungsmittel in kosmetischen und pharmazeutischen Zubereitungen sowie in Nahrungsmitteln eingesetzt.

Es hat jedoch bisher kein Polyglycerinfettsäureestergemisch zur Verfügung gestanden, das als flüssiges, wasserlösliches Produkt vorliegt, einen HLB-Wert über 8 aufweist, so daß es sich zur Herstellung von stabilen O/W-Systemen, insbesondere zur Emulgierung von ätherischen und fetten pflanzlichen oder tierischen Ölen in Badezubereitungen eignet, und eine enge Verteilung hinsichtlich der eingesetzten Glycerinoligomeren aufweist.

Überraschenderweise wurde nun gefunden, daß ein Badezusatzpräparat, insbesondere ein Ölbadpräparat, das gemäß Patentanspruch 1 dadurch gekennzeichnet ist, daß es
- 10 bis 60 Gew.-%: eines Polyglycerinfettsäureestergemisches als wasserlöslichen Emulgator und/oder Solubilisator,
- 10 bis 60 Gew.-%: eines kosmetisch und/oder therapeutisch wirkenden Öls, Ölgemisches und/oder Ölkomponente und
- 0 bis 70 Gew.-%: Wasser
enthält, wobei das Polyglycerinfettsäureestergemisch (bezogen auf 100 Gewichtsteile des Polyglycerinfettsäureestergemisches) einen Anteil von
- 20 bis 65 Gew.-%: Triglycerinfettsäureestern,
- 20 bis 50 Gew.-%: Tetraglycerinfettsäureestern und
- 5 bis 40 Gew.-%: höheren Polyglycerinfettsäureestern
aufweist und keine oder nur geringe Mengen (weniger als 5 Gew.-%) an Diglycerinfettsäureestern enthält und die Fettsäurekomponente aus einer oder mehreren Fettsäuren, ausgewählt aus den gesättigten und/oder ungesättigten, verzweigten und/oder unverzweigten C₆- bis C₁₄-Fettsäuren, mit einem Anteil unter 10 Gew.-% an Fettsäuren mit mehr als 14 C-Atomen, besteht,
alle Anforderungen, die in der Aufgabenstellung genannt wurden, erfüllt. Inbesondere übernimmt das erfindungsgemäß eingesetzte Polyglycerinfettsäureestergemisch eine Doppelfunktion: Einerseits besitzt es einen HLB-Wert über 8 und eignet sich hervorragend zur Emulgierung und Solubilisierung von öligen oder ölhaltigen Stoffen, insbesondere ätherischen und fetten pflanzlichen Ölen, wobei Phasengleichgewichte eingestellt werden können, die den üblichen Kriterien hinsichtlich Stabilität in vollem Umfang genügen.

Die hervorragende emulgierende Wirkung des erfindungsgemäßen Polyglycerinfettsäureestergemisches bleibt auch bei Einarbeitung des erfindungsgemäßen Badezusatzpräparates in stark verdünnte wäßrige Lösungen, wie beispielsweise ein Badewasser, erhalten, wobei sich überraschenderweise gezeigt hat, daß eine Spontanemulgierung der öligen oder ölhaltigen Komponente erfolgt.

Andererseits ist das erfindungsgemäße Polyglycerinfettsäureestergemisch nicht nur physiologisch unbedenklich und biologisch leicht abbaubar, sondern es weist auch einen hautpflegenden und -schützenden, insbesondere einen rückfettenden Effekt auf, der auch bei großer Verdünnung des erfindungsgemäßen Badezusatzpräparates in einem Badewasser zu einem angenehmen Hautgefühl nach dem Bad führt und der Austrockung der Haut durch das Badewasser entgegenwirkt.

Aufgrund dieser Doppelfunktion des Polyglycerinfettsäureestergemisches wird somit die kosmetische Wirksamkeit des erfindungsgemäßen Badezusatzpräparates hinsichtlich Schutz und Pflege der Haut allein schon durch das erfindungsgemäß eingesetzte Polyglycerinfettsäureestergemisch eingestellt, und der Zusatz eines weiteren kosmetisch wirkenden und/oder therapeutisch wirksamem Öls, Ölgemisches und/oder Ölkomponente liefert, je nach eingesetztem Öl, Ölgemisch oder Ölkomponente, einen verstärkenden kosmetischen Effekt und/oder hat eine heilende, lindernde, beruhigende und/oder vitalisierende, physiologische Wirkung.

Daher kann mit dem erfindungsgemäßen Badezusatzpräparat schon unter Verwendung von lediglich zwei Rezepturbestandteilen, nämlich dem erfindungsgemäß eingesetzten Emulgator und einem therapeutisch wirkenden Öl oder Ölgemisch, eine Ölbadformulierung bereitgestellt werden, die einen kosmetischen und einen medizinisch-therapeutschen Effekt aufweist und zudem eine Spontanemulgierung des Öls im Badewasser bewirkt.

In einem mit dem erfindungsgemäßen Badezusatzpräparat hergestellten Wannenbad liegen die Inhaltsstoffe der Ölbadformulierung feinverteilt im Badewasser und/oder auf der Wasseroberfläche (Spreitung) vor und können daher ihre Wirkung optimal entfalten, wobei die mit dem erfindungsgemäßen Polyglycerinfettsäureestergemisch eingestellten Phasengleichgewichte weder durch die in einem Badewasser üblicherweise stark schwankenden Temperaturverhältnisse noch durch im Wasser gelöste Elektrolyte, insbesondere anorganische Salze, gestört werden.

Das erfindungsgemäße Badezusatzpräparat stellt außerdem eine ökologisch sinnvolle Ölbadformulierung dar, denn in dem verbrauchten Badewasser enthaltene Reste des Badezusatzpräparates sind leicht biologisch abbaubar und der erfindungsgemäß eingesetze Emulgator ist zudem physiologisch völlig unbedenklich.

Gemäß einer vorzugsweisen Ausführungsform enthält das erfindungsgemäße Badezusatzpräparat
- 15 bis 50 Gew.-%: eines Polyglycerinfettsäureestergemisches,
- 15 bis 50 Gew.-%: eines kosmetisch und/oder therapeutisch wirkenden Öls, Ölgemisches und/oder Ölkomponente und
- 0,5 bis 60 Gew.-%: Wasser,
wobei das Polyglycerinfettsäureestergemisch (bezogen auf 100 Gewichtsteile des Polyglycerinfettsäureestergemisches) einen Anteil von
- 22 bis 32 Gew.-%: Triglycerinfettsäureestern,
- 39 bis 49 Gew.-%: Tetraglycerinfettsäureestern und
- 24 bis 34 Gew.-%: höheren Polyglycerinfettsäureestern
aufweist und keine oder nur geringe Mengen (weniger als 3 Gew.-%) an Diglycerinfettsäureestern enthält und die Fettsäurekomponente aus einer oder mehreren Fettsäuren, ausgewählt aus den gesättigten und/oder ungesättigten, verzweigten und/oder unverzweigten C₈- bis C₁₂-Fettsäuren, mit einem Anteil unter 5 Gew.-% an Fettsäuren mit mehr als 14 C-Atomen, besteht.

Badezusatzpräparate gemäß der Erfindung enthalten als Fettsäurekomponente in dem erfindungsgemäßen Polyglycerinfettsäureestergemisch vorzugsweise Caprylsäure, Caprinsäure, Laurinsäure, Undecensäure, 2-Ethylhexansäure oder Kokosfettsäure, wobei diese Fettsäuren auch als Gemische untereinander vorliegen können. Durch die vorgenannten Verbindungen können in dem erfindungsgemäßen Polyglycerinfettsäureestergemisch zusätzliche bzw. besondere Eigenschaften eingestellt werden. Dies sind beispielsweise entweder besondere hautpflegende Eigenschaften oder, wie im Fall der Undecensäure, eine antimykotische Wirkung, was für die Konservierung des Badezusatzpräparates von Vorteil ist.

Nach einer weiteren Auführungsform enthält das erfindungsgemäße Badezusatzpräparat als kosmetisch wirkendes Öl fette pflanzliche Öle, vorzugsweise Jojobaöl, Sojaöl, Sesamöl, Erdnußöl, Sonnenblumenöl, Olivenöl, Palmöl, Palmkernöl, Rizinusöl, Kakaoöl, Kokosnußöl, Mandelöl oder Weizenkeimöl, wobei diese Öle allein oder in Mischungen untereinander eingesetzt werden.

Des weiteren kann das erfindungsgemäße Badezusatzpräparat tierische Öle als kosmetisch wirkendes Öl enthalten, vorzugsweise synthetisches Bürzeldrüsenöl, das einen hydrophobierenden Effekt auf der Haut bewirkt.

Mit diesen natürlichen oder naturidentischen Ölen oder Ölgemischen werden in dem erfindungsgemäßen Badezusatzpräparat besondere hautpflegende, -schützende und/oder -regenerierende Wirkungen eingestellt, die die kosmetischen, insbesondere auch rückfettenden Eigenschaften des erfindungsgemäßen Polyglycerinfettsäureestergemisches ergänzen.

Besondere pharmakologische bzw. medizinisch-therapeutische Eigenschaften werden in dem erfindungsgemäßen Badezusatzpräparat durch entsprechend wirkende Öle oder Ölgemische eingestellt, vorzugsweise durch Zusatz von ätherischen Ölen, wie Rosmarinöl, Lavendelöl, Melissenöl, Salbeiöl, Knoblauchöl, Wacholderbeeröl, Anisöl, Kardamonöl, Pimentöl, Anisöl, Kümmelöl, Zitronenöl, Orangenöl, Pfefferminzöl, Campferöl, Nelkenöl, Fichtennadelöl oder Eukalyptusöl, wobei diese Öle allein oder in Mischungen untereinander, eingesetzt werden können.

Sie verleihen dem erfindungsgemäßen Badezusatzpräparat eine medizinische Wirksamkeit, indem sie lindernd bzw. heilend auf den menschlichen Organismus einwirken und/oder ihre therapeutische Wirksamkeit durch einen entspannenden, erfrischenden, vitalisierenden Effekt entfalten.

Gemäß einer weiteren vorzugsweisen Ausführungsform enthält das erfindungsgemäße Badezusatzpräparat als kosmetisch wirkendes Öl und/oder Ölkomponente natürliche oder syntheische Verbindungen, wie Isopropylmyristat, Isopropylpalmitat, Ölsäuredecylester, Cetyl-Stearylisononanoat, 2-Octyldodecanol, Lanolin- oder Cholesterinderivate oder Caprylcaprinsäuretriglycerid, wobei diese Verbindungen allein oder in Mischungen untereinander eingesetzt werden und ebenfalls eine hautpflegende und/oder rückfettende Wirkung haben.

Die vorgenannten natürlichen oder synthetischen, fetten pflanzlichen und tierischen Öle sowie ätherischen Öle und Ölkomponenten können sich gegenseitig ganz oder teilweise in dem erfindungsgemäßen Badezusatzpräparat ersetzen. Sie können aber auch durch pharmazeutisch bzw. therapeutisch wirkende Mineralöle oder -ölgemische, wie beispielsweis Paraffinöl, ganz oder teilweise in dem erfindungsgemäßen Badezusatzpräparat ersetzt sein.

Ein zusätzlicher parfümierender Effekt kann in dem erfindungsgemäßen Badezusatzpräparat durch die Zugabe bestimmter ätherischer Öle, vorzugsweise Rosenöl, Jasminöl, Veilchenöl, Mimosenöl, Orangenöl, Neroliöl, Patchouliöl, Sandelholzöl oder Zimtöl sowie durch Zusatz synthetischer oder natürlicher Parfümkompositionen eingestellt werden, wobei diese Öle oder Parfümkompositionen allein oder in Mischungen untereinander eingesetzt werden. Man erhält dann ein sogenanntes Duftbad.

Als weitere therapeutisch wirksame Substanzen können Lösungen pflanzlicher Extrakte, wie solche der Kamille, in dem Badezusatzpräparat enthalten sein, um beispielsweise entzündliche Prozesse auf der Haut und in den Atemwegsorganen zu lindern bzw. zu heilen.

Das erfindungsgemäße Badezusatzpräparat kann auch als Schaumbad formuliert werden. Hierzu werden zusätzlich schaumaktive Mittel eingearbeitet, wie beispielsweise anionische Tenside, vorzugsweise Alkylethersulfonate und -sulfate, insbesondere Natriumlaurylethersulfat, um ein gutes Schäumvermögen auch bei Fettbelastung zu erhalten.

Als Lösungsmittel kann das erfindungsgemäße Badezusatzpräparat, vorzugsweise entmineralisiertes, Wasser enthalten, das gegebenenfalls geringe Mengen an wasserlöslichen organischen Lösungsmitteln, die gesundheitlich unbedenklich sind, aufweist. Dies sind beispielsweise Glycerin und/oder niedere Alkohole, wie 1,2-Propandiol, die als zusätzliche Lösungsvermittler eingesetzt werden können und Trübungen durch Ausflockung von organischen Bestandteilen in dem Badezusatzpräparat verhindern.

Der Einsatz geringer Wassermengen als Lösungsmittel ist insbesondere dann vorteilhaft, wenn das erfindungsgemäße Badezusatzpräparat aus emulgierendem bzw. solubilisierendem Polyglycerinfettsäureestergemisch und der öligen oder ölhaltigen Komponente eine trübe Lösung bildet. Durch den Wasserzusatz erhält man dann eine optisch klare Lösung.

Inbesondere hinsichtlich seines ausgezeichneten Solubilisierungsvermögens in wasserhaltigen Ölbadpräparaten zeigt das erfindungsgemäße Polyglycerinfettsäureestergemisch seine Überlegenheit gegenüber handelsüblichen Produkten.

Das erfindungsgemäße Polyglycerinfettsäureestergemisch kann zusätzlich mit einem weiteren Polyglycerinfettsäureestergemisch als Lösungsvermittler kombiniert werden. Hierzu eignet sich insbesondere ein Polyglycerincaprinat oder ein Polyglycerincocoat, die vorzugsweise gemäß der eigenen, nicht vorveröffentlichten Patentanmeldung P 41 05 305.2 hergestellt sind.

Bei einem höheren Anteil an pflanzlichen Ölen, insbesondere in einer wasserhaltigen Zubereitung, kann es vorteilhaft sein, daß erfindungsgemäße Badezusatzpräparat gegen mikrobiellen Verderb zu schützen. Als bakteriostatische oder bakterizide Konservierungsmittel können Verbindungen wie Benzoate, Benzoesäurederivate, Sorbate, mikrobiologisch wirksame Phenole, wie 2,6-Di-tert.-butyl-4-methylphenol, und Dioxane, wie 5-Brom-5-nitro-1,3-dioxan, eingesetzt werden.

Gegen eine oxidative Zersetzung kann das erfindungsgemäße Badezusatzpräparat mit Antioxidationsmitteln, wie Tocopherolen, insbesondere Vitamin E, und/oder Butylhydroxytoluol, ausgestattet sein.

Die Konservierungsmittel werden gemäß üblichen Mengen in dem erfindungsgemäßen Badezusatzpräparat eingesetzt.

Als weitere Zusatz- oder Hilfsstoffe können pH-Wert-Regulatoren, Verdicker bzw. Viskositätsregulierungsmittel, wie Polyglykole, Propylenglykol, Ethanol, Isopropanol und/oder anorganische Salze, vorzugsweise Natriumchlorid, Komplexierungsmittel zur Maskierung von Metallionen sowie Farbstoffe in dem erfindungsgemäßen Badezusatzpräparat enthalten sein.

Der pH-Wert des Badezusatzpräparates liegt je nach Wassergehalt vorzugsweise zwischen 5,5 und 7,5.

Das erfindungsgemäße Badezusatzpräparat entfaltet seine vorteilhafte Wirkung insbesondere in einer Menge von 15 bis 30 ml auf etwa 200 Liter Badewasser.

Die Herstellung des erfindungsgemäßen Badezusatzpräparates erfolgt durch einfaches Vermischen der Bestandteile in entsprechenden Mischvorrichtungen, wobei die Reihenfolge der Zugabe der einzelnen Komponenten beliebig ist.

Das erfindungsgemäße Polyglycerinfettsäureestergemisch wird durch Modifizierung des Verfahrens gemäß der eigenen, nicht vorveröffentlichten Patentanmeldung P 42 23 407.7 erhalten, wobei das vorgenannte Verfahren in der Weise modifiziert ist, daß ein Polyglycerin, das (bezogen auf 100 Gew.-teile Polyglycerin) einen Anteil von
- 20 bis 65 Gew.-%: Triglycerin,
- 20 bis 50 Gew.-%: Tetraglycerin und
- 5 bis 40 Gew.-%: höheren Polyglycerinen
aufweist und keine oder nur geringe Mengen (weniger als 5 Gew.-%) an Diglycerin enthält, mit einer oder mehreren Fettsäuren, ausgewählt aus den gesättigten und/oder ungesättigten, verzweigten und/oder unverzweigten C₆- bis C₁₄-Fettsäuren, wobei die Fettsäure oder das Fettsäuregemisch einen Anteil von unter 10 Gew.-% an Fettsäuren mit mehr als 14 C-Atomen aufweist, in einem Molverhältnis des Polyglycerins zu der Fettsäure oder dem Fettsäuregemisch von
4 : 1 bis 1 : 1
gemäß Patentanmeldung P 42 23 407.7 verestert und das erhaltene Polyglycerinfettsäureestergemisch gegebenenfalls aufgearbeitet wird.

Vorzugsweise wird ein Polyglycerin, das (bezogen auf 100 Gew.-teile Polyglycerin) einen Anteil von
- 22 bis 32 Gew.-%: Triglycerin,
- 39 bis 49 Gew.-%: Tetraglycerin und
- 24 bis 34 Gew.-%: höheren Polyglycerinen
aufweist und keine oder nur geringe Mengen (weniger als 3 Gew.-%) an Diglycerin enthält, mit einer oder mehreren Fettsäuren, ausgewählt aus den gesättigten und/oder ungesättigten, verzweigten und/oder unverzweigten C₈- bis C₁₂-Fettsäuren, wobei die Fettsäure oder das Fettsäuregemisch einen Anteil von unter 5 Gew.-% an Fettsäuren mit mehr als 14 C-Atomen aufweist, in einem Molverhältnis des Polyglycerins zu der Fettsäure oder dem Fettsäuregemisch von
2,5 : 1 bis 1,5 : 1, vorzugsweise
2 : 1,
gemäß Patentanmeldung P 42 23 407.7 verestert und das erhaltene Polyglycerinfettsäureestergemisch gegebenenfalls aufgearbeitet.

Die nachfolgenden Ausführungsbeispiele sollen die Erfindung erläutern, ohne sie darauf zu beschränken.

### 1. Ausführungsbeispiel:

### Fichtennadelölbadpräparat, rückfettend

- 45 Gew.-%: erfindungsgemäßes Polyglycerincaprylcaprinat
- 20 Gew.-%: Fichtennadelöl
- 35 Gew.-%: Wasser (vollentsalzt)

### 2. Ausführungsbeispiel:

### Ölbadpräparat mit hoher Pflegewirkung, spreitend

- 20 Gew.-%: erfindungsgemäßes Polyglycerincaprinat
- 20 Gew.-%: Polyglycerincaprinat (hergestellt gemäß P 41 05 305.2)
- 20 Gew.-%: Isopropylmyristat
- 31 Gew.-%: Paraffinoel DAB dickfl.
- 5 Gew.-%: Parfümoel
- 4 Gew.-%: Wasser (vollentsalzt)

### 3. Ausführungsbeispiel:

### pflegendes Ölbadpräparat, spontanemulgierend

- 25 Gew.-%: erfindungsgemäßes Polyglycerincaprinat
- 17 Gew.-%: Polyglycerincocoat (hergestellt gemäß P 41 05 305.2)
- 33 Gew.-%: Isopropylmyristat
- 20 Gew.-%: Jojobaöl
- 5 Gew.-%: Parfümöl

### 4. Ausführungsbeispiel:

### Duftbadpräparat, spontanemulgierend

- 45 Gew.-%: erfindungsgemäßes Polyglycerincaprinat
- 10 Gew.-%: Isopropylmyristat
- 10 Gew.-%: Parfümöl ("Creme Bouquet" der Firma Frey & Lau)
- 35 Gew.-%: Wasser (vollentsalzt)

### 5. Ausführungsbeispiel:

### Ölschaumbadpräparat, wasserlöslich

- 20 Gew.-%: erfindungsgemäßes Polyglycerincaprinat
- 10 Gew.-%: Isopropylmyristat
- 50 Gew.-%: Natriumlaurylethersulfat (28 % Aktivgeh.)
- 10 Gew.-%: Rosmarinöl
- 5 Gew.-%: 1,2-Propandiol
- 5 Gew.-%: Wasser (vollentsalzt)

### 6. Ausführungsbeispiel:

### Ölschaumbadpräparat, wasserlöslich

- 20 Gew.-%: erfindungsgemäßes Polyglycerincaprylcaprinat
- 10 Gew.-%: Isopropylmyristat
- 50 Gew.-%: Natriumlaurylethersulfat (28 % Aktivgeh.)
- 10 Gew.-%: Lavendelöl
- 5 Gew.-%: 1,2-Propandiol
- 5 Gew.-%: Wasser (vollentsalzt)

### 7. Ausführungsbeispiel:

### pflegendes Ölbadpräparat, spontanemulgierend

- 55 Gew.-%: erfindungsgemäßes Polyglycerincaprinat
- 2 Gew.-%: Polyglycerincocoat (hergestellt gemäß P 41 05 305.2)
- 13 Gew.-%: Isopropylmyristat
- 12 Gew.-%: Wacholderbeeröl
- 18 Gew.-%: Wasser (vollentsalzt)

## Patentansprüche

1. Badezusatzpräparat, insbesondere Ölbadpräparat, enthaltend mindestens eine grenzflächenaktive Komponente, vorzugsweise einen wasserlöslichen Emulgator, mindestens eine ölige oder ölhaltige Komponente, ausgewählt aus den natürlichen und synthetischen, mineralischen, ätherischen und fetten tierischen und pflanzlichen Ölen, gegebenenfalls ein Lösemittel oder Lösemittelgemisch sowie gegebenenfalls weitere Hilfs- und Zusatzstoffe, dadurch gekennzeichnet, daß es
10 bis 60 Gew.-% eines Polyglycerinfettsäureestergemisches als wasserlöslichen Emulgator und/oder Solübilisator,
10 bis 60 Gew.-% eines kosmetisch und/oder therapeutisch wirkenden Öls, Ölgemisches und/oder Ölkomponente und
0 bis 70 Gew.-% Wasser
enthält, wobei das Polyglycerinfettsäureestergemisch (bezogen auf 100 Gewichtsteile des Polyglycerinfettsäureestergemisches) einen Anteil von
20 bis 65 Gew.-% Triglycerinfettsäureestern,
20 bis 50 Gew.-% Tetraglycerinfettsäureestern und
5 bis 40 Gew.-% höheren Polyglycerinfettsäureestern
aufweist und keine oder nur weniger als 5 Gew.-% an Diglycerinfettsäureestern enthält und die Fettsäurekomponente aus einer oder mehreren Fettsäuren, ausgewählt aus den gesättigten und/oder ungesättigten, verzweigten und/oder unverzweigten C₆- bis C₁₄-Fettsäuren, mit einem Anteil unter 10 Gew.-% an Fettsäuren mit mehr als 14 C-Atomen, besteht.

2. Badezusatzpräparat nach Anspruch 1, dadurch gekennzeichnet, daß es
15 bis 50 Gew.-% eines Polyglycerinfettsäureestergemisches,
15 bis 50 Gew.-% eines kosmetisch und/oder therapeutisch wirkenden Öls, Ölgemisches und/oder Ölkomponente und
0,5 bis 60 Gew.-% Wasser
enthält, wobei das Polyglycerinfettsäureestergemisch (bezogen auf 100 Gewichtsteile des Polyglycerinfettsäureestergemisches) einen Anteil von
22 bis 32 Gew.-% Triglycerinfettsäureestern,
39 bis 49 Gew.-% Tetraglycerinfettsäureestern und
24 bis 34 Gew.-% höheren Polyglycerinfettsäureestern
aufweist und keine oder nur weniger als 3 Gew.-% an Diglycerinfettsäureestern enthält und die Fettsäurekomponente aus einer oder mehreren Fettsäuren, ausgewählt aus den gesättigten und/oder ungesättigten, verzweigten und/oder unverzweigten C₈- bis C₁₂-Fettsäuren, mit einem Anteil unter 5 Gew.-% an Fettsäuren mit mehr als 14 C-Atomen; besteht.

3. Badezusatzpräparat nach den Ansprüchen 1 oder 2, dadurch gekennzeichnet, daß die Fettsäurekomponente aus Caprylsäure, Caprinsäure, Laurinsäure, Undecensäure, 2-Ethylhexansäure und/oder Kokosfettsäure besteht.

4. Badezusatzpräparat nach einem oder mehreren der Ansprüche 1 bis 3, dadurch gekennzeichnet, daß es als kosmetisch wirkendes Öl Jojobaöl, Sojaöl, Sesamöl, Erdnußöl, Sonnenblumenöl, Olivenöl, Rizinusöl, Palmöl, Palmkernöl, Kakaoöl, Kokosnußöl, Mandelöl oder Weizenkeimöl, allein oder in Mischungen untereinander, enthält.

5. Badezusatzpräparat nach einem oder mehreren der AnAnsprüche 1 bis 4, dadurch gekennzeichnet, daß es als therapeutisch wirkendes Öl ein ätherisches Öl, vorzugsweise Rosmarinöl, Lavendelöl, Melissenöl, Salbeiöl, Knoblauchöl, Wacholderbeeröl, Anisöl, Kardamonöl, Pimentöl, Anisöl, Kümmelöl, Zitro- nenöl, Orangenöl, Pfefferminzöl, Campferöl, Nelkenöl, Fichtennadelöl oder Eukalyptusöl, allein oder in Mischungen untereinander, enthält.

6. Badezusatzpräparat nach einem oder mehreren der Ansprüche 1 bis 5, dadurch gekennzeichnet, daß es als kosmetisch wirkendes Öl und/oder Ölkomponente Isopropylmyristat, Isopropylpalmitat, Ölsäuredecylester, Cetyl-Stearylisononanoat, 2-Octyldodecanol, Lanolin- oder Cholesterinderivate oder Capryl-caprinsäuretriglycerid, allein oder in Mischungen untereinander, enthält.

7. Badezusatzpräparat nach einem oder mehreren der Ansprüche 1 bis 6, dadurch gekennzeichnet, daß es zusätzlich als parfümierende Komponente Rosenöl, Jasminöl, Veilchenöl, Mimosenöl, Orangenöl, Neroliöl, Patchouliöl, Sandelholzöl und/oder Zimtöl und/oder eine Parfümkomposition enthält.

## Claims

1. Bath additive preparation, in particular oil bath preparation, containing at least one surface-active component, preferably a water-soluble emulsifier, at least one oily or oil-containing component selected from the natural and synthetic, mineral, ethereal and fatty animal and vegetable oils, if appropriate a solvent or solvent mixture and if appropriate further auxiliaries and additives, characterised in that it contains
10 to 60 % by weight of a polyglycerol fatty acid ester mixture as the watersoluble emulsifier and/or solubiliser,
10 to 60 % by weight of an oil, oil mixture and/or oil component which has cosmetic and/or therapeutic activity and
0 to 70 % by weight of water,
the polyglycerol fatty acid ester mixture (relative to 100 parts by weight of the polyglycerol fatty acid ester mixture) having a content of
10 to 65 % by weight of triglycerol fatty acid esters,
20 to 50 % by weight of tetraglycerol fatty acid esters
and 5 to 40 % by weight of higher polyglycerol fatty acid esters
and containing no or only less than 5 % by weight of diglycerol fatty acid esters and the fatty acid component consisting of one or more fatty acids, selected from the saturated and/or unsaturated, branched and/or unbranched C₆- to C₁₄-fatty acids, having a content of less than 10 % by weight of fatty acids having more than 14 C atoms.

2. Bath additive preparation according to Claim 1, characterised in that it contains
15 to 50 % by weight of a polyglycerol fatty acid ester mixture,
15 to 50 % by weight of an oil, oil mixture and/or oil component which has a cosmetic and/or therapeutic effect and
0.5 to 60 % by weight of water,
the polyglycerol fatty acid ester mixture (relative to 100 parts by weight of the polyglycerol fatty acid ester mixture) having a content of
22 to 32 % by weight of triglycerol fatty acid esters,
39 to 49 % by weight of tetraglycerol fatty acid esters
and 24 to 34 % by weight of higher polyglycerol fatty acid esters
and containing no or only less than 3 % by weight of diglycerol fatty acid esters and the fatty acid component consisting of one or more fatty acids, selected from the saturated and/or unsaturated, branched and/or unbranched C₈- to C₁₂-fatty acids, having a content of less than 5 % by weight of fatty acids having more than 14 C atoms.

3. Bath additive preparation according to Claims 1 or 2, characterised in that the fatty acid component consists of caprylic acid, capric acid, lauric acid, undecenoic acid, 2-ethylhexanoic acid and/or coconut fatty acid.

4. Bath additive preparation according to one or more of Claims 1 to 3, characterised in that it contains, as the oil having a cosmetic effect, jojoba oil, soya oil, sesame oil, groundnut oil, sunflower oil, olive oil, cator oil, palm oil, palm kernes oil, cocoa oil, coconut oil, almond oil or wheat-germ oil, on their own or in mixtures with one another.

5. Bath additive preparation according to one or more of Claims 1 to 4, characterised in that it contains, as the oil having a therapeutic effect, an ethereal oil, preferably rosemary oil, lavender oil, balm mit oil, sage oil, garlic oil, jiniper berry oil, aniseed oil, cardamon oil, pimento oil, aniseed oil, caraway oil, lemon oil, orange oil, peppermint oil, camphor oil, clove oil, pine-needle oil or eucalyptus oil, on their own or in mixtures with one another.

6. Bath additive preparation according to one or more of Claims 1 to 5, characterised in that it contains, as the oil and/or oil component having cosmetic activity, isopropyl myristate, isopropyl palmitate, decyl oleate, cetyl stearyl isononanoate, 2-octyldodecanol, lanolin or cholesterol derivatives or caprylic-capric acid triglyceride on their own or in mixtures with one another.

7. Bath additive preparation according to one or more of Claims 1 to 6, characterised in that it additionally contains, as the perfuming component, rose oil, jasmine oil, violet oil, mimosa oil, orange oil, neroli oil, patchouli oil, sandalwood oil and/or cinnamon oil and/or a perfume composition.

## Revendications

1. Préparation d'additif pour bain, notamment préparation huileuse pour bain, contenant au moins un constituant tensioactif, avantageusement un émulsifiant hydrosoluble, au moins un constituant huileux ou contenant de l'huile ou de l'essence, choisi parmi les huiles et essences naturelles et synthétiques, minérales, animales et végétales éthérées et grasses, éventuellement un solvant ou un mélange de solvants ainsi que, éventuellement, d'autres adjuvants et additifs, préparation caractérisée en ce qu'elle contient:
10 à 60% en poids d'un mélange d'esters polyglycéroliques d'acides gras comme émulsifiant hydrosoluble et/ou comme solubilisant,
10 à 60% en poids d'une huile, ou essence, ou d'un mélange d'huiles ou d'essences, et/ou d'un constituant huileux à action cosmétique et/ou thérapeutique, et
0 à 70% en poids d'eau,
le mélange d'esters polyglycéroliques d'acides gras présentant (par rapport à 100 parties en poids du mélange d'esters polyglycéroliques d'acides gras) une proportion de:
20 à 65% en poids d'esters triglycéroliques d'acides gras,
20 à 50% en poids d'esters tétraglycéroliques d'acides gras, et
5 à 40% d'esters polyglycéroliques supérieurs d'acides gras,
et pas d'esters diglycéroliques d'acides gras ou moins de 5% en poids seulement d'esters diglycéroliques d'acides gras et le constituant acides gras consistant en un ou plusieurs acides gras choisi(s) parmi les acides gras en C₆ à C₁₄, saturés et/ou insaturés, ramifiés et/ou non ramifiés, avec une proportion inférieure à 10% en poids d'acides gras ayant plus de 14 atomes de carbone.

2. Préparation d'additif pour bain selon la revendication 1, caractérisée en ce qu'elle contient:
15 à 50% en poids d'un mélange d'esters polyglycéroliques d'acides gras
15 à 50% en poids d'une huile ou essence, d'un mélange d'huiles ou d'essences et/ou d'un constituant huileux à action cosmétique et/ou thérapeutique, et
0,5 à 60% en poids d'eau
le mélange des esters polyglycéroliques d'acides gras présentant (par rapport à 100 parties en poids du mélange d'esters polyglycéroliques d'acides gras) une proportion de:
22 à 32% en poids d'esters triglycéroliques d'acides gras,
39 à 49% en poids d'esters tétraglycéroliques d'acides gras, et
24 à 34% en poids d'esters polyglycéroliques supérieurs d'acides gras,
et pas d'esters diglycéroliques d'acides gras ou moins de 3% en poids seulement d'esters diglycéroliques d'acides gras et le constituant acides gras consistant en un ou plusieurs acides gras, choisis parmi les acides gras en C₈ à C₁₂, saturés et/ou insaturés, ramifiés et/ou non ramifiés, avec une proportion inférieure à 5% en poids d'acides gras ayant plus de 14 atomes de carbone.

3. Préparation d'additif pour bain selon les revendications 1 ou 2, caractérisée en ce que le constituant acides gras consiste en de l'acide caprylique, de l'acide caprique, de l'acide laurique, de l'acide undécénoïque, de l'acide 2-éthylhexanoïque et/ou de l'acide gras de coco.

4. Préparation d'additif pour bain selon une ou plusieurs des revendications 1 à 3, caractérisée en ce qu'elle contient, comme huile à action cosmétique, de l'huile de jojoba, de l'huile de soja, de l'huile de sésame, de l'huile d'arachide, de l'huile de tournesol, de l'huile d'olive, de l'huile de ricin, de l'huile de palme, de l'huile de noyau de palme, de l'huile de cacao, de l'huile de noix de coco, de l'huile d'amande, ou de l'huile de germe de blé, seule ou en mélange les unes avec les autres.

5. Préparation d'additif pour bain selon une ou plusieurs des revendications 1 à 4, caractérisée en ce qu'elle contient comme huile ou essence à action thérapeutique, une huile ou essence éthérée, avantageusement de l'essence de romarin, de l'essence de lavande, de l'essence de mélisse, de l'essence de sauge, de l'essence d'ail, de l'essence de genièvre, de l'essence d'anis, de l'essence de cardamone, de l'essence de piment, de l'anisol, de l'essence de cumin, de l'essence de citron, de l'essence d'orange, de l'essence de menthe poivrée, de l'essence de camphre, de l'essence de girofle ou huile d'oeillette, de l'essence d'aiguilles de pin ou de l'essence d'eucalyptus, isolément ou en des mélanges les uns avec les autres.

6. Préparation d'additif pour bain selon l'une ou plusieurs des revendications 1 à 5, caractérisée en ce qu'elle contient, comme huile, essence et/ou constituant huileux à rôle cosmétique, du myristate d'isopropyle, du palmitate d'isopropyle, de l'ester décylique de l'acide oléique, de l'isononanoate de cétyle et de stéaryle, du 2-octyldodécanol, des dérivés de la lanoline ou du cholestérol ou du triglycéride d'acide caprylique-caprique, isolément ou en mélange les uns avec les autres.

7. Préparation d'additif pour bain selon une ou plusieurs des revendications 1 à 6, caractérisée en ce qu'elle contient en outre, comme constituant à rôle de parfum, de l'essence de rose, de l'essence de jasmin, de l'essence de violette, de l'essence de mimosa, de l'essence d'orange, de l'essence de néroli, de l'essence de patchouli, de l'essence de bois de santal et/ou de l'essence de cannelle et/ou une composition de parfums.
